(19) Europäisches Patentamt / European Patent Office / Office européen des brevets

(11) **EP 3 006 423 B1**

(12) **EUROPEAN PATENT SPECIFICATION**

(45) Date of publication and mention of the grant of the patent:
**21.10.2020 Bulletin 2020/43**

(51) Int Cl.:
*C07C 57/03* (2006.01)  *C07C 51/083* (2006.01)
*C04B 24/26* (2006.01)  *C04B 40/00* (2006.01)
*C04B 28/02* (2006.01)  *C04B 103/40* (2006.01)
*C04B 103/30* (2006.01)

(21) Application number: **14804154.4**

(22) Date of filing: **18.04.2014**

(86) International application number:
**PCT/KR2014/003390**

(87) International publication number:
**WO 2014/193082 (04.12.2014 Gazette 2014/49)**

(54) **CEMENT ADMIXTURE COMPRISING POLYCARBOXYLIC ACID COPOLYMER, DERIVED FROM A MACROMONOMER, AND LAYERED DOUBLE HYDROXIDE AND METHOD FOR PREPARING SAME**

ZEMENTZUSATZMITTEL MIT EINEM POLYCARBOXYLSÄURE-COPOLYMER AUS EINEM MAKROMONOMER UND SCHICHT-DOPPELHYDROXID SOWIE VERFAHREN ZUR HERSTELLUNG DAVON

ADJUVANT POUR CIMENT COMPRENANT UN COPOLYMÈRE D'ACIDE POLYCARBOXYLIQUE, DÉRIVÉ D'UN MACROMONOMÈRE, ET HYDROXYDE DOUBLE LAMELLAIRE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2013 KR 20130061194**
**26.07.2013 KR 20130088990**

(43) Date of publication of application:
**13.04.2016 Bulletin 2016/15**

(73) Proprietor: **Silkroad C&T Co., LTD**
**Seoul 137-070 (KR)**

(72) Inventors:
• **KIM, Bo Seung**
**Pyeongtaek-si**
**Gyeonggi-do 450-765 (KR)**
• **KIM, Su Il**
**Seoul 151-892 (KR)**
• **KIM, Jung Sun**
**Seoul 156-773 (KR)**
• **PARK, Kwang Young**
**Yongin-si**
**Gyeonggi-do 446-783 (KR)**
• **CHA, Cheol Yong**
**Hwaseong-si**
**Gyeonggi-do 445-856 (KR)**

(74) Representative: **Böhm, Brigitte**
**Weickmann & Weickmann**
**Patent- und Rechtsanwälte PartmbB**
**Postfach 860 820**
**81635 München (DE)**

(56) References cited:
**EP-A2- 2 412 689**  **WO-A2-2005/075529**
**KR-A- 19980 064 529**  **KR-A- 20060 106 708**
**KR-A- 20070 028 310**  **KR-A- 20080 084 973**
**KR-A- 20120 035 563**

• **PLANK J ET AL: "Preparation and characterization of new Ca-Al-polycarboxylate layered double hydroxides", MATERIALS LETTERS, NORTH HOLLAND PUBLISHING COMPANY. AMSTERDAM, NL, vol. 60, no. 29-30, 7 April 2006 (2006-04-07), pages 3614-3617, XP027898360, ISSN: 0167-577X [retrieved on 2006-12-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- Takehiko Ishii ET AL: "New Synthesis of Heterobifunctional Poly(ethylene glycol) Possessing a Pyridyl Disulfide at One End and a Carboxylic Acid at the Other End", Polymer Journal, 1 January 2005 (2005-01-01), pages 221-228, XP055435668, DOI: 10.1295/polymj.37.221 Retrieved from the Internet: URL:https://www.nature.com/articles/pj2005 30.pdf

## Description

TECHNICAL FIELD

The inventive concept relates to a cement admixture including a polycarboxylic acid-based copolymer derived from a macromonomer and a layered double hydroxide, and a method of preparing the same. More particularly, the inventive concept relates to a macromonomer used to prepare the inventive cement admixture having excellent dispersibility and workability maintenance, and a method of preparing the same. Also, the inventive concept relates to a cement admixture including a polycarboxylic acid-based copolymer derived from the macromonomer and a layered double hydroxide, and a method of preparing the same.

BACKGROUND ART

A cement admixture is a material added to concrete while mixing cement, aggregates, water, and the like, to provide a specific performance thereto. Cement admixtures are classified into mineral admixtures and chemical admixtures, and the chemical admixtures are mainly classified into air-entraining (AE) admixtures, water-reducing admixtures, and high-performance water-reducing admixtures.

Conventionally, melamine-based or naphthalene-based condensates are mainly used as the high-performance water-reducing admixtures. However, since a polycarboxylic acid (PCA)-based admixture was developed in the 1990s, use of PCA-based admixtures has been increasing due to excellent dispersibility obtainable even when a smaller amount than the conventional naphthalene-based water-reducing admixture is used, excellent workability maintenance even with a high water-reducing rate, and high capability of being modified allowing the PCA-based admixtures to be formed in various types by controlling molecular structures thereof.

The PCA-based admixtures may form a three-dimensional molecular structure due to a polyethylene oxide side chain contained in the polymer strucure, and steric hindrance repulsion caused thereby disperses cement particles, thereby fluidizing a cement composition. However, dispersibility of the cement particles decreases with time due to secondary aggregation of the cement particles, a decrease in the repulsion, and the like, thereby deteriorating workability. To solve this problem, when the workability maintenance of a PCA-based admixture is improved by modifying a molecular structure of the polymer, dispersibility may be considerably reduced. On the contrary, when dispersibility of the PCA-based admixture, which is still unsatisfactory, is improved, workability maintenance therof may be considerably reduced.

As described above, since dispersibility and workability maintenance of the PCA-based admixture is in a trade-off relationship, it is very difficult to improve both dispersibility and workability maintenance, simultaneously. Thus, when they are used, it has been widely used that a dispersant-type PCA-based admixture and a workability maintenance-type PCA-based admixture, which have been separately prepared, are mixed together in an appropriate ratio.

Meanwhile, with the costal reclamation and construction of marine architectural buildings, the construction of marine concrete structures has been vigorously performed. In addition, there is a need to use washed sand (sea sand), land sand, and the like as well as river sand to sufficiently obtain aggregates. However, with the construction of marine concrete structures and use of washed sand, chloride ions infiltrate into the concrete structures, thereby causing corrosion in the concrete structures.

PCA-based admixtures, naphthalene-based admixtures, and melamine-based admixtures that have been conventionally used as cement admixtures to date are not suitable for preventing corrosion of the concrete structures due to insufficient corrosion resistance. In addition, although a method of adding an inorganic anti-corrosion agent to a concrete composition has been suggested, the inorganic anti-corrosion agent deteriorates the workability of concrete or generates gas after pouring the concrete, thereby deteriorating the concrete structure. Heterobifunctional poly(ethylene glycol) possessing a pyridyl disulfide at one end and y-carboxylic acid at the other end have been described in a different context by Ishii et al., Polymer Journal, Vol. 37, No. 3, 221-228 (2005). The preparation and characterization of certain Ca-Al-polycarboxylic layered double hydroxides has been disclosed by Plank et al. in Materials Letters, Vol. 60, No 29-30, 3614-2617 (2006).

Korean Patent No. 1195825 (EP 2 412 689 A2) discloses a method of preparing a cement admixture by mixing a polyurethane-based copolymer and a layered double hydroxide, and this method has provided significant corrosion resistance. However, a manufacturing process of this cement admixture includes preparing a urethane compound via an addition reaction between a diisocyanate derivative and a diol, preparing an unsaturated (meth)polyoxyalkylene urethane compound via a reaction between the urethane compound and an unsaturated organic acid or an unsaturated alcohol, and preparing a polyurethane copolymer by polymerizing the unsaturated (meth)polyoxyalkylene urethane compound with an unsaturated anionic organic monomer. Subsequently, the polyurethane copolymer is mixed with a layered double hydroxide. As such, the manufacturing process of the cement admixture includes a variety of complicated stages, most of which require stringent processing conditions such as a nitrogen atmosphere, thereby increasing manufacturing costs therefor. Furthermore, while the cement admixture has excellent corrosion resistance, the dispersibility and workability maintenance thereof are poor, and thus, there is a need to improve these properties.

## DETAILED DESCRIPTION OF THE INVENTIVE CONCEPT

### TECHNICAL PROBLEM

The inventive concept provides a cement admixture that is easily prepared and has excellent dispersibility, workability maintenance, and corrosion resistance.

The inventive concept also provides a method of preparing the cement admixture.

### TECHNICAL SOLUTION

The present invention is defined by the appended claims. Within the context of the inventive concept, described, but not according to the invention, is a macromonomer compound represented by Formula 1 below:

$$[\text{Formula 1}] \qquad CH_2=CR^1-(CH_2)_m-O-(CH_2CH_2O)_n-CO-R^2-COOH$$

$R^1$ is a hydrogen atom or a methyl group; $R^2$ is a C2-C10 alkylene group or a C2-C10 alkenylene group, the alkylene or alkenylene group may be substituted with a C1-C3 alkyl group or a hydroxyl group; m is an integer from 0 to 5; and n, which is an average number of added moles of the oxyethylene group, is an integer from 2 to 150.

According to an exemplary embodiment, the $R^2$ may be a C2-C5 alkylene group or a C2-C5 alkenylene group, wherein the alkylene or alkenylene group may be substituted with a C1-C3 alkyl group or a hydroxyl group.

$R^2$ may be a C2-C5 alkylene group, the alkylene group may be substituted with a C1-C3 alkyl group or a hydroxyl group.

$R^2$ may be substituted with a methyl group or a hydroxyl group.

m may be an integer from 1 to 3; and the n, which is an average number of added moles of the oxyethylene group, may be an integer from 7 to 100.

Also disclosed is a method of preparing a macromonomer compound for preparing a cement admixture, the method including preparing the macromonomer compound represented by Formula 1 by reacting (a) a compound represented by Formula 2 below with (b) a saturated or unsaturated dicarboxylic acid represented by Formula 3, an anhydride thereof, or a mixture thereof:

$$[\text{Formula 2}] \qquad CH_2=CR^1-(CH_2)_m-O-(CH_2CH_2O)_n-H$$

wherein in Formula 2, $R^1$ is a hydrogen atom or a methyl group; m is an integer from 0 to 5; n, which is an average number of added moles of the oxyethylene group, is an integer from 2 to 150; and

$$[\text{Formula 3}] \qquad HOOC-R^2-COOH$$

in Formula 3, $R^2$ is a C2-C10 alkylene group or a C2-C10 alkenylene group, the alkylene or alkenylene group may be substituted with a C1-C3 alkyl group or a hydroxyl group.

The component (b) may be the anhydride of saturated or unsaturated dicarboxylic acid represented by Formula 3, and the reaction of the component (a) with the component (b) may be performed while stirring at a temperature of 55 to 70°C.

The component (b) may be the anhydride of saturated or unsaturated dicarboxylic acid represented by Formula 3, and the reaction of the component (a) with the component (b) may be performed in the absence of a solvent and an additive under atmospheric conditions while stirring at a temperature of 55 to 70°C.

The component (b) may be the saturated or unsaturated dicarboxylic acid represented by Formula 3, and the reaction of the component (a) with the component (b) may be performed in the presence of a polymerization inhibitor at a temperature of 75 to 95°C.

The polymerization inhibitor may include at least one selected from the group consisting of a quinone-based polymerization inhibitor, an alkylphenol-based polymerization inhibitor, an amine-based polymerization inhibitor, an N-oxyl-based polymerization inhibitor, and a copper dithiocarbamate-based polymerization inhibitor.

According to the inventive concept, there is provided a cement admixture including:

(A) a polycarboxylic acid-based copolymer including (a) a structural unit derived from a macromonomer compound represented by Formula 1 below and (b) a structural unit derived from an acrylic monomer represented by Formula 4 below; and (B) a layered double hydroxide represented by Formula 5 below and having a layered structure:

[Formula 1]  $CH_2=CR^1-(CH_2)_mO-(CH_2CH_2O)_n-CO-R^2-COOH$

wherein in Formula 1, $R^1$, $R^2$, m, and n are as defined above;

[Formula 4]  $CH_2=CR^3-CO-M^1$

in Formula 4, $R^3$ is a hydrogen atom or a methyl group; and $M^1$ is $-OM^2$ or $-N(M^2)_2$; $M^2$, each occurrence of which may be are the same or different, is a hydrogen atom or a C1-C3 alkyl group; and

[Formula 5]  $[M^{2+}_{1-x}N^{3+}_x(OH)_2][A^{n-}]_{x/n} \cdot yH_2O$

in Formula 5, $M^{2+}$ is a divalent metal cation; $N^{3+}$ is a trivalent metal cation; A is an anionic chemical species that is ionic-bonded between layers of the layered structure of the hydroxide and has a charge number of n; x is a number that is greater than 0 and less than 1; and y is a positive number that is greater than 0.

According to an exemplary embodiment, in Formula 1, $R^2$ may be a C2-C5 alkylene group or a C2-C5 alkenylene group.

According to another exemplary embodiment, in Formula 1, m may be an integer from 1 to 3, and n may be an integer from 7 to 100.

According to another exemplary embodiment, the structural unit (b) may include two or more structural units derived from the two or more acrylic monomers represented by Formula 4 above.

According to another exemplary embodiment, in at least one of the two or more acrylic monomers represented by Formula 4, $M^1$ may be $-OM^2$.

According to another exemplary embodiment, the two or more acrylic monomers represented by Formula 4 above may include at least one acrylic monomer represented by Formula 4 in which $M^1$ is $-OM^2$ and at least one acrylic monomer represented by Formula 4 in which $M^1$ is $-N(M^2)_2$.

According to another exemplary embodiment, a molar ratio of the structural unit (a) to the structural unit (b) may be in a range of 10:90 to 30:70.

According to another exemplary embodiment, the polycarboxylic acid-based copolymer (A) may have a weight average molecular weight (Mw) of 5,000 to 300,000.

According to another exemplary embodiment, $M^{2+}$ may be selected from the group consisting of $Mg^{2+}$, $Ca^{2+}$, $Co^{2+}$, $Cu^{2+}$, $Ni^{2+}$, and $Zn^{2+}$, $N^{3+}$ may be selected from the group consisting of $Al^{3+}$, $Cr^{3+}$, $Fe^{3+}$, $Ga^{3+}$, $In^{3+}$, $V^{3+}$, and $Ti^{3+}$, and $A^{n-}$ may be selected from the group consisting of $NO_2^-$, $NO_3^-$, $CO_3^{2-}$, $OH^-$, $O^{2-}$, $SO_4^{2-}$, a halide, a metalate, and an organic acid anion.

According to another exemplary embodiment, the amount of the polycarboxylic acid-based copolymer (A) may be in a range of 40 to 95% by weight and the amount of the layered double hydroxide (B) may be in a range of 5 to 60% by weight based on a total weight of the cement admixture.

According to another aspect of the inventive concept, there is provided a method of preparing a cement admixture, the method including: (I) preparing a macromonomer compound represented by Formula 1 below by reacting (a1) a compound represented by Formula 2 below with (a2) a saturated or unsaturated dicarboxylic acid represented by Formula 3 below, an anhydride thereof, or a mixture thereof; (II) preparing (A) a polycarboxylic acid-based copolymer by reacting (a) the macromonomer compound with (b) an acrylic monomer represented by Formula 4 below; (III) preparing (B) a layered double hydroxide represented by Formula 5 below and having a layered structure; and (IV) reacting (A) the polycarboxylic acid-based copolymer with (B) the layered double hydroxide:

[Formula 1]  $CH_2=CR^1-(CH_2)_m-O-(CH_2CH_2O)_n-CO-R^2-COOH$

[Formula 2]  $CH_2=CR^1-(CH_2)_m-O-(CH_2CH_2O)_n-H$

[Formula 3]  $HOOC-R^2-COOH$

[Formula 4]  $CH_2=CR^3-CO-M^1$

[Formula 5]  $[M^{2+}_{1-x}N^{3+}_x(OH)_2][A^{n-}]_{x/n} \cdot yH_2O$

wherein in Formulae 1 to 5, each substituent is as defined above.

According to an exemplary embodiment, in step (I), the component (a2) may be the anhydride of saturated or unsaturated dicarboxylic acid represented by Formula 3, and step (I) may be performed in the absence of a solvent and an additive under atmospheric conditions while stirring at a temperature of 55 to 70°C.

According to another exemplary embodiment, in step (I), the component (a2) may be the saturated or unsaturated dicarboxylic acid represented by Formula 3, and step (I) may be performed in the presence of a polymerization inhibitor while stirring at a temperature of 75 to 95°C.

According to another exemplary embodiment, step (II) may be performed in the presence of a polymerization initiator, and the polymerization initiator may be a peroxide-based initiator.

According to another exemplary embodiment, step (IV) may be performed at a temperature less than 50°C.

ADVANTAGEOUS EFFECTS

A cement admixture having excellent dispersibility and workability maintenance may be prepared using a macromonomer compound represented by Formula 1 according to the inventive concept.

The cement admixture according to the inventive concept is prepared by modifying a polycarboxylic acid-based copolymer used in conventional PCA-based admixtures and mixing the modified polycarboxylic acid-based copolymer with a layered double hydroxide, and thus, dispersibility or workability maintenance may be improved while maintaining excellent corrosion resistance. The cement admixture is economical and efficient since it is simply and easily manufactured.

DESCRIPTION OF THE DRAWINGS

FIG. 1 is a $^1$H-NMR spectrum of a monomer prepared according to Example 1;
FIG. 2 is a $^1$H-NMR spectrum of a monomer prepared according to Example 2;
FIG. 3 is a $^1$H-NMR spectrum of a monomer prepared according to Example 3; and
FIG. 4 is a $^1$H-NMR spectrum of a monomer prepared according to Example 4.

BEST MODE

As used herein, the terms "alkylene" and "alkenylene" may include a linear or branched alkylene and a linear or branched alkenylene, respectively.

As used herein, the expression "in the absence of an additive" refers to not using any additives, such as a reaction initiator, and a reaction assisting agent, other than reactants contributing to a chemical structure of the final product.

As used herein, the term "cement admixture" refers collectively to any material that is added to cement, mortar, or a concrete composition to provide a specific performance thereto.

Hereinafter, a monomer for preparing a cement admixture according to embodiments of the inventive concept will be described in detail.

The monomer for preparing a cement admixture according to the is a compound represented by Formula 1 below:

[Formula 1]        $CH_2=CR^1-(CH_2)_m-O-(CH_2CH_2O)_n-CO-R^2-COOH$

In Formula 1, $R^1$ is a hydrogen atom or a methyl group, preferably a methyl group.

In Formula 1, $R^2$ is a C2-C10 alkylene group or a C2-C10 alkenylene group, preferably a C2-C5 alkylene group or a C2-C5 alkenylene group, more preferably a C2-C3 alkylene group or a C2-C3 alkenylene group. Particularly, $R^2$ may be an alkylene group, i.e., a C2-C10 alkylene group, more preferably a C2-C5 alkylene group, and most preferably a C2-C3 alkylene group. In this regard, the alkylene or alkenylene defined as $R^2$ may be substituted with a C1-C3 alkyl group or a hydroxyl group, preferably with a methyl group or a hydroxyl group, more preferably with a methyl group, and most preferably may not be substituted.

In Formula 1, m is an integer from 0 to 5, preferably an integer from 1 to 3.

In Formula 1, n, which is an average number of added moles of the oxyethylene group, is an integer from 2 to 150, preferably an integer from 7 to 100. When n is greater than 150, polymerization ability of monomers may be reduced. When n is less than 2, steric hindrance that is sufficient for dispersing cement particles may not be obtained and excellent flowability may not be obtained.

Hereafter, a method of preparing the monomer for preparing a cement admixture according to embodiments of the inventive concept will be described in detail.

The method of preparing the monomer for preparing a cement admixture of the inventive concept includes preparing a compound represented by Formula 1 above by reacting (a) a compound represented by Formula 2 below with (b) a saturated or unsaturated dicarboxylic acid represented by Formula 3, an anhydride thereof, or a mixture thereof:

[Formula 2]     $CH_2=CR^1-(CH_2)_m-O-(CH_2CH_2O)_n-H$

[Formula 3]     $HOOC-R^2-COOH$

In Formulae 2 and 3, $R^1$, $R^2$, m, and n are as defined above with reference to Formula 1 above.

Examples of the component (b) may be succinic acid, glutaric acid, maleic acid, fumaric acid, methyl glutaric acid, malic acid, and an anhydride thereof.

In the method, when the component (b) is an anhydride of the saturated or unsaturated dicarboxylic acid, the reaction of the component (a) with the component (b) may be performed while stirring at a temperature of 55 to 70°C. The reaction temperature may preferably be in a range of 55 to 65°C. The reaction may be performed in the absence of a solvent and an additive under atmospheric conditions, and thus is very economical and efficient.

In the method, when the component (b) is a saturated or unsaturated dicarboxylic acid, the reaction of the component (a) with the component (b) may be performed in the presence of a polymerization inhibitor while stirring at a temperature of 75 to 95°C. The reaction temperature may preferably be in a range of 80 to 90°C. The reaction step may be performed in the presence of a solvent under atmospheric conditions.

The polymerization inhibitor may include the following polymerization inhibitors: a quinone-based polymerization inhibitor, such as hydroquinone, methoxy hydroquinone, benzoquinone, and p-tert-butyl catechol; an alkylphenol-based polymerization inhibitor, such as 2,6-di-tert-butylphenol, 2,4-di-tert-butylphenol, 2-tert-butyl-4,6-dimethylphenol, 2,6-di-tert-butyl-4-methylphenol, and 2,4,6-tri-tert-butylphenol; an amine-based polymerization inhibitor, such as an alkylated diphenylamine, N,N'-diphenyl-p-phenylenediamine, and phenothiazine; an N-oxyl-based polymerization inhibitor, such as 4-hydroxy-2,2,6,6-tetramethylpiperidine-N-oxyl; and a copper dithiocarbamate-based polymerization inhibitor, such as copper dimethyldithiocarbamate, copper diethyldithiocarbamate, and copper dibutyldithiocarbamate. These may be used alone or in a combination of at least two chemical species. Among them, the quinone-based polymerization inhibitor and the N-oxyl-based polymerization inhibitor may preferably be used, and hydroquinone, methoxy hydroquinone, benzoquinone, p-tert-butyl catechol, phenothiazine, and 4-hydroxy-2,2,6,6-tetramethylpiperidine-N-oxyl may be suitable. The amount of the polymerization inhibitor may be appropriately adjusted according to types of the component (a). However, the amount thereof may be in a range of 0.001 to 5 parts by weight, preferably 0.005 to 1 parts by weight, more preferably 0.01 to 0.1 parts by weight, based on 100 parts by weight of the component (a) by taking into account polymerization inhibition effects, yield, productivity, and cost efficiency.

As the solvent, the following solvents may be used alone or in a combination of at least two chemical species thereof: an aromatic hydrocarbon, such as benzene, toluene, and xylene; an aliphatic hydrocarbon, such as pentane, hexane, cyclohexane, and heptane; an ether, such as diethyl ether and diisopropyl ether; a ketone, such as acetone and methylethyl ketone; a polar solvent, such as dimethyl formamide and dimethyl sulfoxide; a halogenated hydrocarbon, such as chloroform, methylene chloride, dichloroethane, and chlorobenzene; ethylene glycol dimethyl ether, ethylene glycol diethyl ether, ethylene glycol dipropyl ether, propylene glycol dimethyl ether, propylene glycol diethyl ether, diethylene glycol dimethyl ether, and diethylene glycol diethyl ether; ethylene glycol monomethyl ether acetate, ethylene glycol monoethyl ether acetate, ethylene glycol monopropyl ether acetate, ethylene glycol monobutyl ether acetate, ethylene glycol monophenyl ether acetate, diethylene glycol monomethyl ether acetate, diethylene glycol monoethyl ether acetate, diethylene glycol monopropyl ether acetate, diethylene glycol monobutyl ether acetate, diethylene glycol monophenyl ether acetate, propylene glycol monomethyl ether acetate, dipropylene glycol monoethyl ether acetate, propylene glycol monopropyl ether acetate, 2-methoxybutyl acetate, 3-methoxybutyl acetate, 4-methoxybutyl acetate, 2-methyl-3-methoxybutyl acetate, 3-methyl-3-methoxybutyl acetate, 3-ethyl-3-methoxybutyl acetate, 2-ethoxybutyl acetate, 4-ethoxybutyl acetate, 4-propoxybutyl acetate, 2-methoxypentyl acetate, 3-methoxypentyl acetate, 4-methoxypentyl acetate, 2-methyl-3-methoxypentyl acetate, 3-methyl-3-methoxypentyl acetate, 3-methyl-4-methoxypentyl acetate, 4-methyl-4-methoxypentyl acetate, dimethyl glutarate, dimethyl succinate, and dimethyl adipate; acetone, methyl ethyl ketone, diethyl ketone, methyl isobutyl ketone, ethyl isobutyl ketone, tetrahydrofuran, cyclohexanone, methyl propionate, ethyl propionate, propyl propionate, isopropyl propionate, methyl-3-methoxy propionate, ethyl-3-methoxy propionate, ethyl-3-ethoxy propionate, ethyl-3-propoxy propionate, propyl-3-methoxypropionate, isopropyl-3-methoxy propionate, methyl lactate, ethyl lactate, propyl lactate, isopropyl lactate, butyl lactate, amyl lactate, ethyl ethoxyacetate, ethyl oxyacetate, methyl acetate, ethyl acetate, propyl acetate, isopropyl acetate, butyl acetate, isoamyl acetate, methyl carbonate, ethyl carbonate, propyl carbonate, butyl carbonate, methyl pyruvate, ethyl pyruvate, propyl pyruvate, butyl pyruvate, methyl acetoacetate, ethyl acetoacetate, benzyl methyl ether, benzyl ethyl ether, dihexyl ether, benzyl acetate, ethyl benzoate, diethyl oxalate, diethyl malate, and γ-butyrolactone. The solvent may be added in an amount of about 5 to about 15% by weight, preferably about 10% by weight, based on the total weight of reactants.

Polycarboxylic acid (PCA)-based admixtures, both dispersant-type products and workability maintenance-type products, have common features in that they are prepared by polymerizing an unsaturated monomer including a polyoxyalkylene unit and a monocarboxylic acid-based unsaturated monomer. The macromonomer compound represented by Formula 1 is an unsaturated monomer including a polyoxyalkylene unit. The macromonomer compound is polymerized with a monocarboxylic acid-based unsaturated monomer to form a copolymer, and the copolymer is used to form a cement admixture. The monocarboxylic acid-based unsaturated monomer and the polymerization method are not particularly limited.

Hereafter, (A) a polycarboxylic acid-based copolymer including (a) a structural unit derived from the macromonomer compound and (b) a structural unit derived from an acrylic monomer; and (B) a layered double hydroxide included in the cement admixture according to the inventive concept will be described in detail.

The macromonomer compound for forming the structural unit (a) of the inventive concept is a compound represented by Formula 1 below:

$$[Formula\ 1] \qquad CH_2=CR^1\text{-}(CH_2)_m\text{-}O\text{-}(CH_2CH_2O)_n\text{-}CO\text{-}R^2\text{-}COOH$$

In Formula 1, $R^1$ is a hydrogen atom or a methyl group, preferably a methyl group.

In Formula 1, $R^2$ is a C2-C10 alkylene group or a C2-C10 alkenylene group, preferably a C2-C5 alkylene group or a C2-C5 alkenylene group, more preferably a C2-C3 alkylene group or a C2-C3 alkenylene group. Particularly, $R^2$ may be an alkylene group, i.e., a C2-C10 alkylene group, more preferably a C2-C5 alkylene group, and most preferably a C2-C3 alkylene group. In this regard, the alkylene group or alkenylene group defined as $R^2$ may be substituted with a C1-C3 alkyl group or a hydroxyl group, preferably with a methyl group or a hydroxyl group, more preferably with a methyl group, and most preferably may not be substituted.

In Formula 1, m is an integer from 0 to 5, preferably an integer from 1 to 3.

In Formula 1, n, which is an average number of added moles of the oxyethylene group, is an integer from 2 to 150, preferably an integer from 7 to 100. When n is greater than 150, polymerization ability may be reduced. When n is less than 2, excellent flowability may not be obtained.

The acrylic monomer used to form the structural unit (b) used herein is a compound represented by Formula 4 below:

$$[Formula\ 4] \qquad CH_2=CR^3\text{-}CO\text{-}M^1$$

In Formula 4, $R^3$ is a hydrogen atom or a methyl group.

In Formula 4, $M^1$ is $-OM^2$ or $-N(M^2)_2$, preferably $-OM^2$ or $-NH(M^2)$. Here, $M^2$, each occurrence of which may be the same or different, is a hydrogen atom or a C1-C3 alkyl group, preferably a hydrogen atom or a methyl group.

The structural unit (b) may include two or more structural units derived from two or more acrylic monomers represented by Formula 4 above. Preferably, at least one $M^1$ is $-OM^2$ in the two or more acrylic monomers represented by Formula 4. Particularly, the two or more structural units (b) may include two or more acrylic monomers represented by Formula 4 in which $M^1$ is $-OM^2$, or may include at least one acrylic monomer represented by Formula 4 in which $M^1$ is $-OM^2$ and at least one acrylic monomer represented by Formula 4 in which $M^1$ is $-N(M^2)_2$. For example, the structural unit (b) may include two structural units derived from the two acrylic monomers represented by Formula 4 in which $M^1$ is $-OM^2$, in which all of the two $M^2$ may be hydrogen atoms (in this case, $R^3$ is a hydrogen atom in one acrylic monomer, and $R^3$ is a methyl group in the other acrylic monomer), or one $M^2$ may be a hydrogen atom and the other $M^2$ may be a C1-C3 alkyl group. As another examples, the structural unit (b) may include two structural units including one structural unit derived from an acrylic monomer represented by Formula 4 in which $M^1$ is $-OM^2$ and the other structural unit derived from an acrylic monomer represented by Formula 4 in which $M^1$ is $-NH(M^2)$.

When the structural unit (b) includes two or more structural units (b) derived from two or more acrylic monomers represented by Formula 4, a composition ratio of each of the acrylic monomers is not particularly limited. However, for example, when two acrylic monomers are used, a composition ratio thereof may be 90:10 to 10:90, preferably 80:20 to 20:80, more preferably 75:25 to 25:75 in a molar ratio.

A molar ratio of the structural unit (a) to the structural unit (b) may be in a range of 10:90 to 30:70, preferably 15:85 to 25:75.

A weight average molecular weight of the polycarboxylic acid-based copolymer (A) including the structural units (a) and (b) according to the inventive concept is not particularly limited, but may be in a range of 5,000 to 300,000, preferably 7,000 to 100,000, more preferably 9,000 to 80,000, and most preferably 10,000 to 70,000. When the weight average molecular weight is within the ranges above, the cement admixture may have excellent dispersibility and workability.

The cement admixture according to the present inventive concept may include the polycarboxylic acid-based copolymer (A) alone or in a mixture of at least two thereof. In addition, the cement admixture according to the inventive concept may further include any other known polymer for the cement admixture, such as a conventional polycarboxylic acid-based polymer, a naphthalene-based polymer, a melamine-based polymer, and a polyurethane-based polymer, in addition to the polycarboxylic acid-based copolymer (A).

The layered double hydroxide (B) used herein is a compound represented by Formula 5 below:

[Formula 5] $\qquad [M^{2+}_{1-x}N^{3+}_{x}(OH)_2][A^{n-}]_{x/n} \cdot yH_2O$

In Formula 5, $M^{2+}$ is a divalent metal cation. For example, $M^{2+}$ may be selected from the group consisting of $Mg^{2+}$, $Ca^{2+}$, $Co^{2+}$, $Cu^{2+}$, $Ni^{2+}$, and $Zn^{2+}$. $N^{3+}$ is a trivalent metal cation. For example, $N^{3+}$ may be selected from the group consisting of $Al^{3+}$, $Cr^{3+}$, $Fe^{3+}$, $Ga^{3+}$, $In^{3+}$, $V^{3+}$, and $Ti^{3+}$. A is an anionic chemical species that is ionic-bonded between layers of the layered structure of the layered double hydroxide and has a charge number of n. For example, $A^{n-}$ may be selected from the group consisting of $NO_2^-$, $NO_3^-$, $CO_3^{2-}$, $OH^-$, $O^{2-}$, $SO_4^{2-}$, a halide, a metalate, and an organic acid anion, preferably $NO_2^-$. X is greater than 0 and less than 1, and y is a positive number that is greater than 0.

The layered double hydroxide (B) may have an interlayer spacing of about 7 to about 9 Å and a size of about 300 to about 500 nm.

In addition, in the cement admixture according to the inventive concept, the polycarboxylic acid-based copolymer (A) and the layered double hydroxide (B) may be used in amounts of 40 to 95% by weight and 5 to 60% by weight, respectively, based on the total weight of the cement admixture. When the amount of the layered double hydroxide (B) is 60% by weight or less, the cement admixture may have excellent dispersibility and workability maintenance. Meanwhile, in order to prevent phase separation of the polycarboxylic acid-based copolymer (A) and the layered double hydroxide (B), the layered double hydroxide (B) may be added in an amount of 50% by weight or less. Accordingly, the polycarboxylic acid-based copolymer (A) and the layered double hydroxide (B) may be included in amounts of 50 to 95% by weight and 5 to 50% by weight, preferably 50 to 90% by weight and 10 to 50% by weight, respectively, based on the total weight of the cement admixture. If a phase separation between the polycarboxylic acid-based copolymer (A) and the layered double hydroxide (B) occurs, the phase separation may be removed by stirring while or after adding the cement admixture to the concrete composition.

A method of preparing the cement admixture, according to the inventive concept, includes the following steps:

(I) preparing a macromonomer compound represented by Formula 1 above by reacting (a1) a compound represented by Formula 2 below with (a2) a saturated or unsaturated dicarboxylic acid represented by Formula 3 below, an anhydride thereof, or a mixture thereof;

(II) preparing (A) a polycarboxylic acid-based copolymer by reacting (a) the macromonomer compound with (b) an acrylic monomer represented by Formula 4 above;

(III) preparing (B) a layered double hydroxide represented by Formula 5 above and having a layered structure; and

(IV) reacting (A) the polycarboxylic acid-based copolymer with (B) the layered double hydroxide:

[Formula 1] $\qquad CH_2=CR^1-(CH_2)_m-O-(CH_2CH_2O)_n-CO-R^2-COOH$

[Formula 2] $\qquad CH_2=CR^1-(CH_2)_m-O-(CH_2CH_2O)_n-H$

[Formula 3] $\qquad HOOC-R^2-COOH$

In Formulae 1 and 2, $R^1$, $R^2$, m, and n are as defined above with reference to Formula 1 above.

In step (I), examples of the component (a2) may include succinic acid, glutaric acid, maleic acid, fumaric acid, methyl glutaric acid, malic acid, and anhydride thereof. The component (a2) may be used approximately in an amount of 1 equivalent (eq.) relative to the component (a1).

In step (I), when the component (a2) is an anhydride of the saturated or unsaturated dicarboxylic acid, step (I) may be performed while stirring at a temperature of 55 to 70°C. The reaction temperature may preferably be 55 to 65°C. Step (I) may be performed in the absence of a solvent and an additive under atmospheric conditions and thus it is economical and efficient.

In step (I), when the component (a2) is a saturated or unsaturated dicarboxylic acid, step (I) may be performed while stirring in the presence of a polymerization inhibitor at a temperature of 75 to 95°C. The reaction temperature may preferably be 80 to 90°C. Step (I) may be performed in the presence of a solvent under atmospheric conditions.

Examples of the polymerization inhibitor may include the followings: a quinone-based polymerization inhibitor, such as hydroquinone, methoxy hydroquinone, benzoquinone, and p-tert-butyl catechol; an alkylphenol-based polymerization inhibitor, such as 2,6-di-tert-butylphenol, 2,4-di-tert-butylphenol, 2-tert-butyl-4,6-dimethylphenol, 2,6-di-tert-butyl-4-methylphenol, and 2,4,6-tri-tert-butylphenol; an amine-based polymerization inhibitor, such as an alkylated diphenylamine, N,N'-diphenyl-p-phenylenediamine, and phenothiazine; an N-oxyl-based polymerization inhibitor, such as 4-hydroxy-2,2,6,6-tetramethylpiperidine-N-oxyl; and a copper dithiocarbamate-based polymerization inhibitor, such as copper dimethyldithiocarbamate, copper diethyldithiocarbamate, and copper dibutyldithiocarbamate. These may be used alone or in a combination of at least two chemical species. Among them, the quinone-based polymerization inhibitor and the N-oxyl-based polymerization inhibitor may preferably be used, and hydroquinone, methoxy hydroquinone, benzoquinone, p-tert-butyl catechol, phenothiazine, and 4-hydroxy-2,2,6,6-tetramethylpiperidine-N-oxyl may be suitable. The amount of the polymerization inhibitor may be appropriately adjusted according to types of the component (a1) and may be in a range of preferably 0.001 to 5 parts by weight, preferably 0.005 to 1 parts by weight, more preferably 0.01 to 0.1 parts by weight, based on 100 parts by weight of the component (a1) by taking into account polymerization inhibition effects, yield, productivity, and cost efficiency.

When the solvent is used in step (I), the solvent may be, but is not limited to, water, an aromatic or aliphatic hydrocarbon such as benzene, toluene, xylene, cyclohexane, and n-hexane, an ether-based compound such as tetrahydrofuran and dioxane, a ketone-based compound such as acetone and methylethyl ketone, an alcohol such as methyl alcohol, ethyl alcohol, and isopropyl alcohol, ethyl acetate, dimethyl formamide, chloroform, and methylene chloride. The solvent may be used alone or in a combination of at least two chemical species. Although the amount of the solvent is not particularly limited so long as it is sufficient for the reactions, the solvent may be added in an amount of about 5 to 15% by weight, preferably about 10% by weight, based on the total weight of the reactants.

In step (II), the macromonomer compound (a) and the acrylic monomer (b) may be used in the same amounts described above with reference to the cement admixture.

Step (II) may be performed under atmospheric conditions. Thus, since stringent conditions such as a nitrogen atmosphere are not required, it is efficient and economical.

In addition, in step (II), water may be used as the solvent, and the amount of the solvent may be determined such that an aqueous solution including all reactants including the macromonomer compound (a) and the acrylic monomer (b) has a concentration of 50%.

In addition, step (II) may be performed in the presence of a polymerization initiator. If required, any other additional component, for example, a chain transfer agent, may further be added to the reaction.

The polymerization initiator may be any known polymerization initiator, for example, a persulfate compound such as ammonium persulfate, sodium persulfate, and potassium persulfate; hydrogen peroxide; an azo compound such as azobis-2-methylpropione amidine hydrochloride and azoisobutyronitrile; and a peroxide compound such as benzoyl peroxide, lauroyl peroxide, cumene hydroperoxide, and t-amyl peroxy-2-ethylhexanoate, preferably a peroxide compound. The polymerization initiator may be used alone or in a combination of at least two chemical species in an amount of 0.05 to 2% by weight, preferably 0.1 to 1% by weight, based on the total weight of all reactants used in the reaction of step (II).

The chain transfer agent may be any known chain transfer agent, for example, a thiol-based chain transfer agent such as mercaptoethanol, thioglycerol, thioglycolic acid, mercaptopropionic acid, 2-mercaptopropionic acid, 3-mercaptopropionic acid, octyl thioglycolate, octyl 3-mercaptopropionate, 2-mercaptoethanesufonic acid, n-dodecyl mercaptan, octyl mercaptan, and butylthioglycolate; a halide such as carbon tetrachloride, carbon tetrabromide, methylene chloride, bromoform, and bromotrichloroethane; an unsaturated hydrocarbon compound such as $\alpha$-methylstylne dimer, $\alpha$-terpinene, dipentene, and terpinolene; a primary alcohol such as 2-aminopropane-1-ol; a secondary alcohol such as isopropanol; phosphorous acid, hypophosphorous acid, and salts thereof; and a sulfite, a bisulfite, a dithionite, a metabisulfite, and salts thereof. The chain transfer agent may be used alone or in a combination of at least two chemical species in an amount of 0.05 to 2% by weight, preferably 0.1 to 1% by weight, based on the total weight of all reactants used in the reaction of step (II).

In step (II), the polymerization temperature and polymerization time may be appropriately selected according to polymerization methods and types of the polymerization initiator and the chain transfer agent. In general, the polymerization temperature may be in a range of 0°C to 180°C, preferably 40°C to 150°C, more preferably 60°C to 120°C. In addition, the polymerization time may be in a range of 0.5 to 24 hours, preferably 1 to 12 hours, more preferably 2 to 10 hours.

Once the polymerization is complete in step (II), the pH may be adjusted to 2.5 to 6.5 by using an appropriate pH adjusting agent. The pH may be in a range of 4 to 6 by taking into account handling feasibility or dispersibility of a cement admixture prepared therefrom. The pH adjusting agent may be a hydroxide of a monovalent metal, a hydroxide of a divalent metal, a carbonate of a monovalent metal, a carbonate of a divalent metal, ammonia, an organic amine, and the like. For example, sodium hydroxide may be used.

Step (II) is preferably performed so that the polycarboxylic acid-based copolymer (A) obtained in step (II) may have a conversion rate of 70% or greater, as measured by gel permeation chromatography (GPC). When the conversion rate is less than 70%, corrosion resistance may not be sufficiently obtained due to ion-exchange between an anion inside of the layered double hydroxide and an unreacted anion of the polycarboxylic acid-based copolymer (A).

In step (III), the layered double hydroxide (B) may be prepared using a known method and typically synthesized by a substitution reaction between a divalent metal and a trivalent metal.

In step (IV), the polycarboxylic acid-based copolymer (A) and the layered double hydroxide (B) may be used in the same amounts as described above with reference to the cement admixture.

Step (IV) may be performed at a temperature less than 50°C. When the temperature is 50°C or greater than, the structure of the layered double hydroxide (B) may be destroyed. The lower limit of the fusion temperature of step (IV) is not particularly limited, but may be 0°C or greater.

MODE OF THE INVENTIVE CONCEPT

Hereinafter, one or more embodiments of the inventive concept will be described in detail with reference to the following examples and comparative examples. These examples and comparative examples are not intended to limit the purpose and scope of the one or more embodiments.

[Preparation of Monomers]

Example 1 (for illustration)

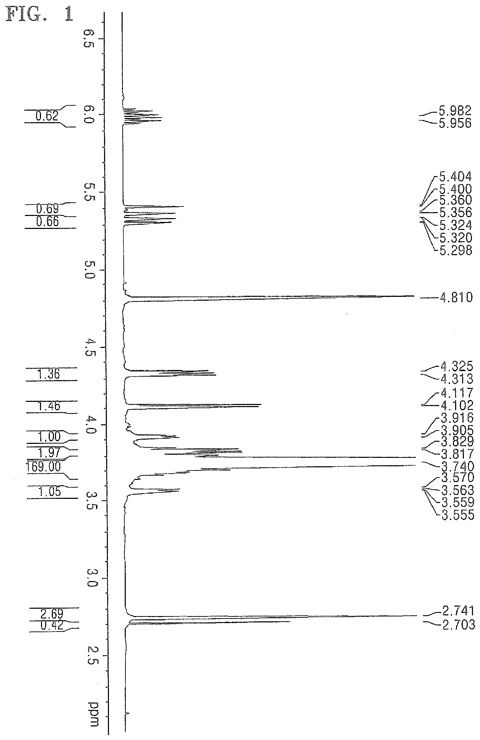

1000 g of a compound represented by the formula $CH_2=CH-CH_2-O-(CH_2CH_2O)_n-H$ (having a weight average molecular weight of 2400) and 41.7 g of succinic anhydride were added to a reactor equipped with a stirrer and a thermometer, and the reactor was slowly heated to 60°C. After being heated to 60°C, the mixture was stirred under atmospheric conditions at 90 rpm for 5 hours to obtain a light yellow clear liquid phase material with a yield of 95%. A $^1$H-NMR spectrum of the obtained monomer is shown in FIG. 1.

Example 2 (for illustration)

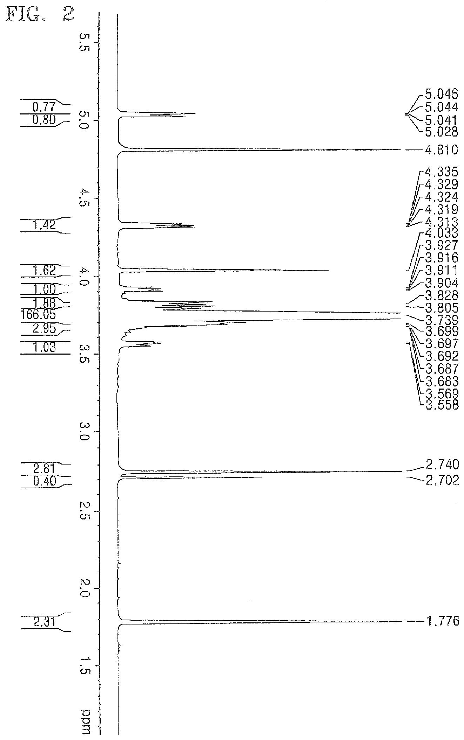

1000 g of a compound represented by the formula $CH_2=C(CH_3)-CH_2-O-(CH_2CH_2O)_n-H$ (having a weight average molecular weight of 2400) and 41.7 g of succinic anhydride were added to a reactor equipped with a stirrer and a thermometer, and the reactor was slowly heated to 60°C. After being heated to 60°C, the mixture was stirred under atmospheric conditions at 90 rpm for 5 hours to obtain a clear liquid phase material with a yield of 90%. A $^1$H-NMR spectrum of the obtained monomer is shown in FIG. 2.

Example 3 (for illustration)

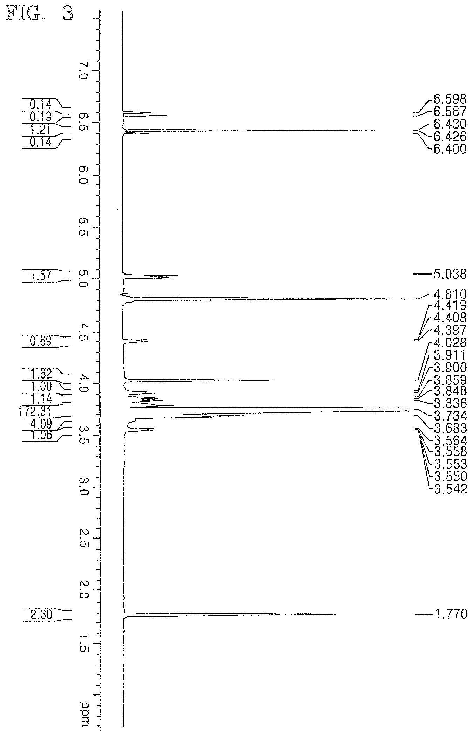

1000 g of a compound represented by the formula $CH_2=C(CH_3)-CH_2-O-(CH_2CH_2O)_n-H$ (having a weight average molecular weight of 2400) and 40.8 g of maleic anhydride were added to a reactor equipped with a stirrer and a thermometer, and the reactor was slowly heated to 60°C. After being heated to 60°C, the mixture was stirred under atmospheric conditions at 90 rpm for 5 hours to obtain a clear liquid phase material with a yield of 50%. A $^1$H-NMR spectrum of the obtained monomer is shown in FIG. 3.

Example 4 (for illustration)

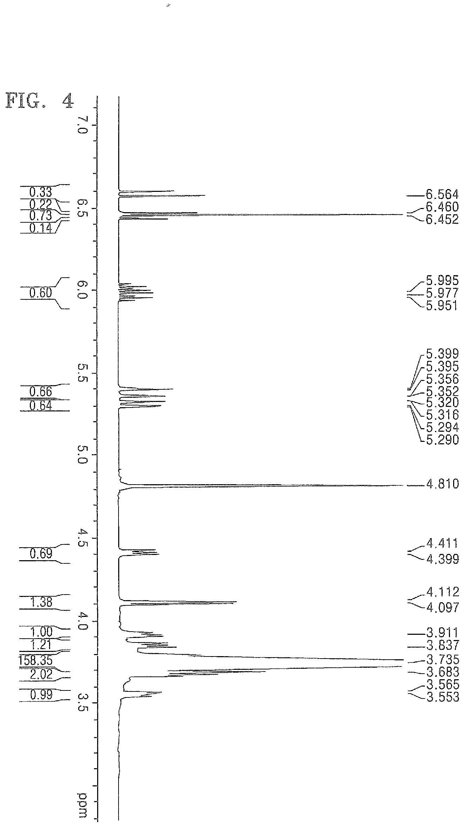

1000 g of a compound represented by the formula $CH_2=CH-CH_2-O-(CH_2CH_2O)_n-H$ (having a weight average molecular weight of 2400) and 40.8 g of maleic anhydride were added to a reactor equipped with a stirrer and a thermometer, and the reactor was slowly heated to 60°C. After being heated to 60°C, the mixture was stirred under atmospheric conditions at 90 rpm for 5 hours to obtain a light yellow clear liquid phase material with a yield of 50%. A $^1$H-NMR spectrum of the obtained monomer is shown in FIG. 4.

[Preparation of Cement Admixture]

Preparation Example 1

15.3% by weight of acrylic acid, 13.4% by weight of methacrylic acid, 0.1% by weight 2-mercaptoethanol as a chain transfer agent, and 1.2% by weight of t-amyl peroxy-2-ethylhexanoate as an organic initiator were slowly added dropwise to 70% by weight of the monomer prepared according to Example 1, and the mixture was stirred at 95°C for 6 hours. Once the reaction was complete, the resulting polymer was cooled to 60°C, and caustic soda was added thereto under atmospheric conditions to adjust the pH of the resultant mixture to 4.5. The polymer was produced with a yield of 73%, and a molecular weight (Mw) of the polymer was 44,000 (viscosity: 410±30 cps).

Preparation Example 2

A polymer was prepared in the same manner as in Preparation Example 1, except that the monomer prepared according to Example 2 instead of Example 1, was used and a hydroperoxide was used as the organic initiator. The polymer was produced with a yield of 76%, and a molecular weight (Mw) of the polymer was 50,000 (viscosity: $450\pm30$ cps).

Comparative Preparation Example 1

A polymer was prepared in the same manner as in Preparation Example 1, except that a compound represented by the formula $CH_2=C(CH_3)-CH_2-O-(CH_2CH_2O)_n-H$ (having a weight average molecular weight of 2400) was used instead of the monomer prepared according to Example 1. The polymer was produced with a yield of 79%, and a molecular weight (Mw) of the polymer was 44,000 (viscosity: $430\pm30$ cps).

[Evaluation of Performance of Concrete]

A cement composition having the ingredients shown in Table 1 was prepared:

[Table 1]

| W/b (%) | S/A (%) | Unit weight (kg/m$^3$) | | | | | | | AD (%) |
|---|---|---|---|---|---|---|---|---|---|
| | | Water | Cement | Slag powder | Fly Ash | Washed sand | Crushed sand | 25 mm coarse aggregate | |
| 45.2 | 46 | 165 | 292 | - | 73 | 461 | 310 | 920 | 0.9 |
| In Table 1, W/b denotes a ratio of water to the binder (i.e., cement+fly ash), S/A denotes a ratio of sand to coarse aggregate, and<br>AD denotes an amount of the cement admixture based on the total weight of the binder. Here, cement available under the trade name of CHEONMAPYO CEMENT from SUNGSHIN | | | | | | | | | |
| CEMENT Co., Ltd. was used as the cement, and Fly ash from Taean Electric Power Plant located in Taean-gun, Chungchengnam-do, Republic of Korea was used as the fly ash. | | | | | | | | | |

The cement composition including the ingredients listed in Table 1 was prepared and mixed to prepare concrete. The concrete was filled in a flow cone having a diameter of 20 cm and a height of 30 cm, and the flow cone was vertically lifted. A diameter of a concrete spread on a flow table was measured in two directions, and an average thereof was used as a flow value (mm). Flow values measured at an early stage of mixing, at 30 minutes after mixing, and at 60 minutes after mixing are shown in Table 2 below.

[Table 2]

| | Flow value (mm) | | | Flowability maintenance (last flow value/first flow value) |
|---|---|---|---|---|
| | Initial | After 30 minutes | After 60 minutes | |
| A case in which the cement admixture of Preparation Example 1 was used | 480 | 460 | 430 | 0.90 |
| A case in which the cement admixture of Preparation Example 2 was used | 520 | 480 | 440 | 0.85 |
| A case in which the cement admixture of Comparative Preparation Example 1 was used | 435 | 407 | 360 | 0.83 |

As shown in Table 2, when the cement admixtures prepared according to Preparation Example 1 and Preparation Example 2 using the monomers according to the inventive concept were used, concretes having improved flowability maintenance (i.e., workability maintenance) and improved initial flowability (i.e., dispersibility) were obtained. As such, improvement in both workability maintenance and dispersibility is a surprising result since it has been known that dispersibility and workability are in a trade-off relationship in PCA-based cement admixtures in which improvement in one property causes considerable deterioration in the other property.

[Preparation of Cement Admixture According to the Inventive Concept]

Example 5

(1) Preparation of Macromonomer Compound represented by Formula 1

1000 g of a compound represented by the formula $CH_2=CH-CH_2-O-(CH_2CH_2O)_n-H$ (having a weight average molecular weight of 2400) was added to a reactor equipped with a thermometer, and 1 eq. of succinic anhydride was added thereto. The reactants were slowly heated to 60°C and stirred under atmospheric conditions at about 90 rpm for 5 hours to obtain a light yellow clear liquid phase material with a yield of 95%.

(2) Preparation of Polycarboxylic acid-based copolymer

A reactor equipped with a thermometer and two dropping funnels was heated to 95°C. A solution prepared by mixing 760.00 g of the obtained macromonomer compound, 65.72 g of acrylic acid, 26.17 g of methacrylic acid, and 0.2% by weight of 2-mercaptoethanol as a chain transfer agent (weight % based on a total weight of the macromonomer compound, acrylic acid, and methacrylic acid as 100% by weight) was added to one dropping funnel, and 0.1% by weight of t-amyl peroxy-2-ethylhexanoate as a polymerization initiator (weight % based on the total weight of the macromonomer compound, acrylic acid, and methacrylic acid as 100% by weight) was added to the other dropping funnel. Then, they were slowly added dropwise to the reactor for 6 hours to prepare a polycarboxylic acid-based copolymer. Once polymerization was complete, the reactor was cooled to 60°C, caustic soda was added thereto to adjust the pH of the mixture to 4.5. The polycarboxylic acid-based copolymer had a conversion rate of 73% measured by GPC and a weight average molecular weight of 44,000. The weight average molecular weight was obtained by a calibration method using a polyethylene glycol standard.

(3) Preparation of Layered Double Hydroxide

$Mg(NO_3)_2 \cdot 6H_2O$ (0.4 M) and $Al(NO_3)_3 \cdot 9H_2O$ (0.2 M) were dissolved in process water, and the pH of the solution was adjusted to 9 to 10 by using a NaOH aqueous solution in which $NaNO_2$ (0.2 M) was dissolved, to obtain a layered metal double hydroxide suspension formed by precipitation. The obtained layered double hydroxide suspension was stirred at 100°C for 12 hours, unreacted salts were removed therefrom by washing, and the resultant was freeze-dried to obtain a layered double hydroxide. The obtained layered double hydroxide has an interlayer spacing of 8 Å, a size of 350 nm, a pH of 11.3, and a viscosity of 180 cps.

(4) Preparation of Cement Admixture

300 g of the obtained layered double hydroxide was allowed to stand in a jacket-type reactor and stirred under atmospheric conditions at about 100 rpm. While stirring, 1,200 g of the obtained polycarboxylic acid-based copolymer was added thereto. In this regard, in order to remove heat of neutralization, the copolymer was slowly added dropwise to the reactor for 2 hours, and the temperature of the reactants was maintained not to exceed 50°C by circulating cooling water from an external cooler. Accordingly, a cement admixture (E-1) having a pH of 5.5 and a viscosity of 340 cps, as a light yellow opaque liquid phase material, was obtained.

Examples 6 and 7

Cement admixtures (E-2 and E-3) were prepared in the same manner as in Example 5, except that acrylamide (21.61 g) and methyl methacrylate (26.17 g) were respectively used instead of methacrylic acid in the preparation of the polycarboxylic acid-based copolymer as shown in Table 3 below.

Example 8

A cement admixture (E-4) was prepared in the same manner as in Example 5, except that a macromonomer compound prepared using maleic anhydride instead of the succinic anhydride, 65.77 g of acrylic acid, and 26.19 g of methacrylic acid were used in the preparation of the polycarboxylic acid-based copolymer.

Examples 9 and 10

Cement admixtures (E-5 and E-6) were prepared in the same manner as in Example 5, except that a macromonomer compound prepared using maleic anhydride instead of the succinic anhydride and 65.77 g of acrylic acid were used, and acrylamide (21.63 g) and methyl methacrylate (26.19 g) were respectively used instead of methacrylic acid as shown in Table 3 below in the preparation of the polycarboxylic acid-based copolymer.

Comparative Example 1

A cement admixture (C-1) was prepared in the same manner as in Example 5, except that a polyurethane copolymer prepared by using an unsaturated poly(meth)oxyethylene urethane compound (a urethane derivative, the number of added moles of the oxyethylene: 45) and methacrylic acid in a molar ratio of 68:32 was used instead of the polycarboxylic acid-based copolymer according to the inventive concept.

[Table 3]

| | Cement admixture No. | Composition ratio of copolymer (mol%) | Conversion rate of copolymer (%) | Weight average molecular weight (Mw) |
|---|---|---|---|---|
| Example 5 | E-1 | PEG-SUC:AA:MAA = 20:60:20 | 73 | 44,000 |
| Example 6 | E-2 | PEG-SUC:AA:AAM = 20:60:20 | 77 | 47,000 |
| Example 7 | E-3 | PEG-SUC:AA:MAM = 20:60:20 | 76 | 46,000 |
| Example 8 | E-4 | PEG-MAH:AA:MAA = 20:60:20 | 73 | 53,000 |
| Example 9 | E-5 | PEG-MAH:AA:AAM = 20:60:20 | 77 | 56,000 |
| Example 10 | E-6 | PEG-MAH:AA:MAM = 20:60:20 | 76 | 55,000 |
| Comparative Example 1 | C-1 | POEU-45:MAA = 68:32 | 75 | 41,000 |

\* PEG-SUC: the macromonomer compound prepared using succinic anhydride,

\* PEG-MAH: the macromonomer compound prepared using maleic anhydride,

\* POEU-45: the unsaturated poly(meth)oxyethylene urethane compound (a urethane derivative, the number of added moles of the oxyethylene : 45)

\* AA: acrylic acid, \* MAA: methacrylic acid,

\* AAM: acrylamide, \* MAM: methyl methacrylate

[Performance Evaluation]

1. Evaluation of Dispersibility and Workability Maintenance

A concrete composition including the ingredients shown in Table 4 below was prepared:

[Table 4]

| Water/ Cement (%) | Sand/Coarse aggregate (%) | Unit weight (kg/m$^3$) | | | | Amount of cement admixture (%) |
|---|---|---|---|---|---|---|
| | | Water | Cement | Sand | Coarse aggregate | |
| 44 | 41 | 176 | 400 | 761 | 1,092 | 0.8 |

The concrete composition including the ingredients listed in Table 4 was prepared and mixed to prepare concrete.

The concrete was filled in a flow cone having a diameter of 20 cm and a height of 30 cm, and the flow cone was vertically lifted. A diameter of a concrete spread on a flow table was measured in two directions, and an average thereof was used as a flow value (mm). Flow values measured at an early stage of mixing and at 30 minutes after mixing are shown in Table 5 below.

[Table 5]

| Cement admixture No. | Flow value (mm) | | Flowability maintenance (last flow value/first flow value) |
|---|---|---|---|
| | Initial | After 30 minutes | |
| E-1 | 550 | 420 | 0.76 |
| E-2 | 500 | 400 | 0.80 |
| E-3 | 520 | 410 | 0.79 |
| E-4 | 470 | 390 | 0.83 |
| E-5 | 450 | 370 | 0.82 |
| E-6 | 490 | 400 | 0.82 |
| C-1 | 470 | 360 | 0.76 |

Referring to Table 5, it was confirmed that initial flowability (i.e., dispersibility) or flowability maintenance (i.e., workability maintenance) may be improved using the cement admixture according to the inventive concept. Particularly, when the polycarboxylic acid-based copolymers including the structural unit derived from the macromonomer compound prepared using succinic anhydride were used in the cement admixtures (E-1 to E-3), dispersibility was significantly improved. When the polycarboxylic acid-based copolymers including the structural unit derived from the macromonomer compound prepared using maleic anhydride were used in the cement admixtures (E-4 to E-6), workability maintenance was significantly improved.

2. Evaluation of Corrosion Resistance

"Accelerated corrosion experiment of reinforcing bar in concrete" was performed using the cement admixtures E-1 and C-1 according to KS F 2561. To calculate anti-corrosion rate, a conventional polycarboxylic acid-based admixture (Product Name: 300 NX, Manufacturer: Silkroad C&T) was used as a plain specimen to which an anti-corrosion agent was not added.

First, a concrete composition including the ingredients listed in Table 6 was prepared:

[Table 6]

| Cement/ Water (%) | Cement (kg/m$^3$) | Unit weight of fine aggregate (kg/m$^3$) | Unit weight (kg/m$^3$) | | | Added amount of cement admixture (%) |
|---|---|---|---|---|---|---|
| | | | Water | Saline solution | Total | |
| 60 | 300 | 800 | 126.5 | 48.5 | 175 | 3 |

The prepared concrete composition was mixed to prepare concrete, and reinforcing bars were inserted thereinto to prepare a specimen. The specimen was added to an autoclave and cured twice at 180°C at 1.0 MPa for 5 hours or more to accelerate corrosion of the reinforcing bars. Then, the reinforcing bars were collected therefrom, and an area of corroded portion was measured, and then an anti-corrosion rate thereof was calculated using the following equation:

$$\text{Anti-corrosion rate (\%)} = 1 - [\text{corrosion area of evaluated specimen /corrosion area of plain specimen}] \times 100$$

The results are shown in Table 7 below.

[Table 7]

| Plain specimen | | E-1 | | C-1 | |
|---|---|---|---|---|---|
| - | | 95% | | 95% | |
| Plain specimen | | E-1 | | C-1 | |

Referring to Table 7, an anti-corrosion rate of the cement admixture E1 according to the inventive concept was 95%, which was the same as that of the conventional corrosion resistance cement admixture C-1. Thus, it was confirmed that the cement admixture according to the inventive concept had improved dispersibility and workability maintenance while maintaining excellent corrosion resistance.

INDUSTRIAL APPLICABILITY

The inventive concept may be efficiently used to prepare a macromonomer for preparing a cement admixture; and a cement admixture including a polycarboxylic acid-based copolymer derived from the macromonomer and a layered double hydroxide.

**Claims**

1.  A cement admixture comprising:

(A) a polycarboxylic acid-based copolymer comprising (a) a structural unit derived from a macromonomer compound represented by Formula 1 below and (b) a structural unit derived from an acrylic monomer represented by Formula 4 below; and
(B) a layered double hydroxide represented by Formula 5 below and having a layered structure:

[Formula 1]        $CH_2=CR^1-(CH_2)_m-O-(CH_2CH_2O)_n-CO-R^2-COOH$

wherein in Formula 1, $R^1$ is a hydrogen atom or a methyl group; $R^2$ is a C2-C10 alkylene group or a C2-C10 alkenylene group, the alkylene or alkenylene group may be substituted with a C1-C3 alkyl group or a hydroxyl group; m is an integer from 0 to 5; n, which is an average number of added moles of the oxyethylene group, is an integer from 2 to 150;

[Formula 4]        $CH_2=CR^3-CO-M^1$

in Formula 4, $R^3$ is a hydrogen atom or a methyl group; and $M^1$ is $-OM^2$ or $-N(M^2)_2$; $M^2$, each occurrence of which may be the same or different, is a hydrogen atom or a C1-C3 alkyl group; and

[Formula 5]        $[M^{2+}_{1-x}N^{3+}_x(OH)_2][A^{n-}]_{x/n} \cdot yH_2O$

in Formula 5, $M^{2+}$ is a divalent metal cation; $N^{3+}$ is a trivalent metal cation; A is an anionic chemical species that is ionic-bonded between layers of the layered structure of the hydroxide and has a charge number of n; x is a number that is greater than 0 and less than 1; and y is a positive number that is greater than 0.

2. The cement admixture of claim 1, wherein the structural unit (b) comprises two or more structural units derived from two or more acrylic monomers represented by Formula 4 above.

3. The cement admixture of claim 2, wherein in at least one of the two or more acrylic monomers represented by Formula 4, $M^1$ is $-OM^2$.

4. The cement admixture of claim 1 or 2, wherein a molar ratio of the structural unit (a) to the structural unit (b) is in a range of 10:90 to 30:70.

5. The cement admixture of claim 1 or 2, wherein $M^{2+}$ is selected from the group consisting of $Mg^{2+}$, $Ca^{2+}$, $Co^{2+}$, $Cu^{2+}$, $Ni^{2+}$, and $Zn^{2+}$, $N^{3+}$ is selected from the group consisting of $Al^{3+}$, $Cr^{3+}$, $Fe^{3+}$, $Ga^{3+}$, $In^{3+}$, $V^{3+}$, and $Ti^{3+}$, and $A^{n-}$ is selected from the group consisting of $NO_2^-$, $NO_3^-$, $CO_3^{2-}$, $OH^-$, $O^{2-}$, $SO_4^{2-}$, a halide, a metalate, and an organic acid anion.

6. The cement admixture of claim 1 or 2, wherein the amount of the polycarboxylic acid-based copolymer (A) is in a range of 40 to 95% by weight and the amount of the layered double hydroxide (B) is in a range of 5 to 60% by weight based on a total weight of the cement admixture.

7. A method of preparing a cement admixture, the method comprising:

(I) preparing (a) a macromonomer compound represented by Formula 1 below by reacting (a1) a compound represented by Formula 2 below with (a2) a saturated or unsaturated dicarboxylic acid represented by Formula 3, an anhydride thereof, or a mixture thereof;
(II) preparing (A) a polycarboxylic acid-based copolymer by reacting (a) the macromonomer compound with (b) an acrylic monomer represented by Formula 4 below;
(III) preparing (B) a layered double hydroxide represented by Formula 5 below and having a layered structure; and
(IV) reacting (A) the polycarboxylic acid-based copolymer with (B) the layered double hydroxide:

[Formula 1] $CH_2=CR^1-(CH_2)_m-O-(CH_2CH_2O)_n-CO-R^2-COOH$

wherein in Formula 1, $R^1$ is a hydrogen atom or a methyl group; $R^2$ is a C2-C10 alkylene group or a C2-C10 alkenylene group, the alkylene or alkenylene group may be substituted with a C1-C3 alkyl group or a hydroxyl group; m is an integer from 0 to 5; and n, which is an average number of added moles of the oxyethylene group, is an integer from 2 to 150;

[Formula 2] $CH_2=CR^1-(CH_2)_m-O-(CH_2CH_2O)_n-H$

in Formula 2, $R^1$, m, and n are as defined above with reference to Formula 1;

[Formula 3] $HOOC-R^2-COOH$

in Formula 3, $R^2$ is as defined above with reference to Formula 1,

[Formula 4] $CH_2=CR^3-CO-M^1$

in Formula 4, $R^3$ is a hydrogen atom or a methyl group; and $M^1$ is $-OM^2$ or $-N(M^2)_2$; and $M^2$, each occurrence of which may be the same or different, is a hydrogen atom or a C1-C3 alkyl group; and

[Formula 5] $[M^{2+}_{1-x}N^{3+}_x(OH)_2][A^{n-}]_{x/n} \cdot yH_2O$

in Formula 5, $M^{2+}$ is a divalent metal cation; $N^{3+}$ is a trivalent metal cation; A is an anionic chemical species that is ionic-bonded between layers of the layered structure of the hydroxide and has a charge number of n; x is a number that is greater than 0 and less than 1; and y is a positive number that is greater than 0.

8. The method of claim 7, wherein in step (I), the component (a2) is the anhydride of saturated or unsaturated dicarboxylic acid represented by Formula 3, and step (I) is performed in the absence of a solvent and an additive under atmospheric conditions while stirring at a temperature of 55 to 70°C.

9. The method of claim 7, wherein in step (I), the component (a2) is the saturated or unsaturated dicarboxylic acid represented by Formula 3, and step (I) is performed in the presence of a polymerization inhibitor while stirring at a temperature of 75 to 95°C.

10. The method of any one of claims 7 to 9, wherein step (II) is performed in the presence of a polymerization initiator, and the polymerization initiator is a peroxide-based initiator.

**Patentansprüche**

1. Zementbeimischung umfassend:

   (A) ein Copolymer auf Polycarbonsäurebasis umfassend (a) eine Struktureinheit, die von einer Makromonomerverbindung, dargestellt durch die nachstehende Formel 1, abgeleitet ist und (b) eine Struktureinheit, die von einem Acrylmonomer, dargestellt durch die nachstehende Formel 4, abgeleitet ist;
   und
   (B) ein geschichtetes Doppelhydroxid, dargestellt durch die nachstehende Formel 5 und mit einer Schichtstruktur:

   $$[\text{Formel 1}] \qquad CH_2=CR^1-(CH_2)_m-O-(CH_2CH_2O)_n-CO-R^2-COOH$$

   wobei in Formel 1 $R^1$ ein Wasserstoffatom oder eine Methylgruppe ist; $R^2$ eine C2-C10-Alkylengruppe oder eine C2-C10-Alkenylengruppe ist, wobei die Alkylen- oder Alkenylengruppe mit einer C1-C3-Alkylgruppe oder einer Hydroxygruppe substituiert sein kann; m eine ganze Zahl von 0 bis 5 ist; n, das eine durchschnittliche Anzahl von zugesetzten Mol der Oxyethylengruppe ist, eine ganze Zahl von 2 bis 150 ist;

   $$[\text{Formel 4}] \qquad CH_2=CR^3-CO-M^1$$

   in Formel 4, $R^3$ ein Wasserstoffatom oder eine Methylgruppe ist; und $M^1$ -$OM^2$ oder -$N(M^2)_2$ ist; $M^2$, dessen jeweiliges Vorkommen gleich oder unterschiedlich sein kann, ein Wasserstoffatom oder eine C1-C3-Alkylgruppe ist; und

   $$[\text{Formel 5}] \qquad [M^{2+}_{1-x}N^{3+}_x(OH)_2][A^{n-}]_{x/n} \cdot yH_2O$$

   in Formel 5, $M^{2+}$ ein zweiwertiges Metallkation ist; $N^{3+}$ ein dreiwertiges Metallkation ist; A eine anionische chemische Spezies ist, die zwischen Schichten der Schichtstruktur des Hydroxids ionisch gebunden ist und eine Ladungszahl von n aufweist; x eine Zahl ist, die größer als 0 und kleiner als 1 ist; und y eine positive Zahl ist, die größer als 0 ist.

2. Zementbeimischung nach Anspruch 1, wobei die Struktureinheit (b) zwei oder mehr Struktureinheiten umfasst, die von zwei oder mehr Acrylmonomeren, dargestellt durch Formel 4, abgeleitet sind.

3. Zementbeimischung nach Anspruch 2, wobei in mindestens einem der zwei oder mehr Acrylmonomere, dargestellt durch Formel 4, $M^1$ -$OM^2$ ist.

4. Zementbeimischung nach Anspruch 1 oder 2, wobei ein Molverhältnis der Struktureinheit (a) zur Struktureinheit (b) in einem Bereich von 10:90 bis 30:70 liegt.

5. Zementbeimischung nach Anspruch 1 oder 2, wobei $M^{2+}$ ausgewählt ist aus der Gruppe bestehend aus $Mg^{2+}$, $Ca^{2+}$, $Co^{2+}$, $Cu^{2+}$, $Ni^{2+}$ und $Zn^{2+}$, $N^{3+}$ ausgewählt ist aus der Gruppe bestehend aus $Al^{3+}$, $Cr^{3+}$, $Fe^{3+}$, $Ga^{3+}$, $In^{3+}$, $V^{3+}$ und $Ti^{3+}$ und $A^{n-}$ ausgewählt ist aus der Gruppe bestehend aus $NO_2^-$, $NO_3^-$, $CO_3^{2-}$, $OH^-$, $O^{2-}$, $SO_4^{2-}$, einem Halogenid, einem Metallat und einem organischen Säureanion.

6. Zementbeimischung nach Anspruch 1 oder 2, wobei die Menge des Copolymers (A) auf Polycarbonsäurebasis in einem Bereich von 40 bis 95 Gew.-% liegt und die Menge des geschichteten Doppelhydroxids (B) in einem Bereich von 5 bis 60 Gew.-% bezogen auf ein Gesamtgewicht der Zementbeimischung liegt.

7. Verfahren zur Herstellung einer Zementbeimischung, wobei das Verfahren umfasst:

(I) Herstellen (a) einer Makromonomerverbindung, dargestellt durch die nachstehende Formel 1, durch Umsetzen (a1) einer Verbindung, dargestellt durch die nachstehende Formel 2, mit (a2) einer gesättigten oder ungesättigten Dicarbonsäure, dargestellt durch Formel 3, einem Anhydrid davon oder einer Mischung davon;

(II) Herstellen (A) eines Copolymers auf Polycarbonsäurebasis durch Umsetzen von (a) der Makromonomerverbindung mit (b) einem Acrylmonomer, dargestellt durch die nachstehende Formel 4;

(III) Herstellen (B) eines geschichteten Doppelhydroxids, dargestellt durch die nachstehende Formel 5 und mit einer Schichtstruktur; und

(IV) Umsetzen von (A) dem Copolymer auf Polycarbonsäurebasis mit (B) dem geschichteten Doppelhydroxid:

[Formel 1] $\qquad$ $CH_2=CR^1-(CH_2)_m-O-(CH_2CH_2O)_n-CO-R^2-COOH$

wobei in Formel 1 $R^1$ ein Wasserstoffatom oder eine Methylgruppe ist; $R^2$ eine C2-C10-Alkylengruppe oder eine C2-C10-Alkenylengruppe ist, wobei die Alkylen- oder Alkenylengruppe mit einer C1-C3-Alkylgruppe oder einer Hydroxygruppe substituiert sein kann; m eine ganze Zahl von 0 bis 5 ist; und n, das eine durchschnittliche Anzahl von zugesetzten Mol der Oxyethylengruppe ist, eine ganze Zahl von 2 bis 150 ist;

[Formel 2] $\qquad$ $CH_2=CR^1-(CH_2)_m-O-(CH_2CH_2O)_n-H$

in Formel 2, $R^1$, m und n wie oben in Bezug auf Formel 1 definiert sind;

[Formel 3] $\qquad$ $HOOC-R^2-COOH$

in Formel 3, $R^2$ wie oben in Bezug auf Formel 1 definiert ist,

[Formel 4] $\qquad$ $CH_2=CR^3-CO-M^1$

in Formel 4, $R^3$ ein Wasserstoffatom oder eine Methylgruppe ist; und $M^1$ $-OM^2$ oder $-N(M^2)_2$ ist; und $M^2$, dessen jeweiliges Vorkommen gleich oder unterschiedlich sein kann, ein Wasserstoffatom oder eine C1-C3-Alkylgruppe ist; und

[Formel 5] $\qquad$ $[M^{2+}_{1-x}N^{3+}_x(OH)_2][A^{n-}]_{x/n} \cdot yH_2O$

in Formel 5, $M^{2+}$ ein zweiwertiges Metallkation ist; $N^{3+}$ ein dreiwertiges Metallkation ist; A eine anionische chemische Spezies ist, die zwischen Schichten der Schichtstruktur des Hydroxids ionisch gebunden ist und eine Ladungszahl von n aufweist; x eine Zahl ist, die größer als 0 und kleiner als 1 ist; und y eine positive Zahl ist, die größer als 0 ist.

8. Verfahren nach Anspruch 7, wobei in Schritt (I) die Komponente (a2) das Anhydrid der gesättigten oder ungesättigten Dicarbonsäure, dargestellt durch Formel 3, ist und Schritt (I) in der Abwesenheit eines Lösungsmittels und eines Additivs unter atmosphärischen Bedingungen unter Rühren bei einer Temperatur von 55 bis 70 °C durchgeführt wird.

9. Verfahren nach Anspruch 7, wobei in Schritt (I) die Komponente (a2) die gesättigte oder ungesättigte Dicarbonsäure, dargestellt durch Formel 3, ist und Schritt (I) in der Anwesenheit eines Polymerisationsinhibitors unter Rühren bei einer Temperatur von 75 bis 95 °C durchgeführt wird.

10. Verfahren nach einem der Ansprüche 7 bis 9, wobei Schritt (II) in der Anwesenheit eines Polymerisationsinitiators durchgeführt wird und der Polymerisationsinitiator ein Initiator auf Peroxidbasis ist.

## Revendications

1. Adjuvant pour ciment comprenant :

(A) un copolymère à base d'acide polycarboxylique comprenant (a) un motif structural dérivé d'un composé macromonomère représenté par la formule 1 ci-dessous et (b) un motif structural dérivé d'un monomère acrylique représenté par la formule 4 ci-dessous ; et

(B) un hydroxyde double lamellaire représenté par la formule 5 ci-dessous et ayant une structure lamellaire :

[Formule 1]     $CH_2=CR^1-(CH_2)_m-O-(CH_2CH_2O)_n-CO-R^2-COOH$

dans lequel dans la formule 1, $R^1$ est un atome d'hydrogène ou un groupe méthyle ; $R^2$ est un groupe alkylène en C2-C10 ou un groupe alcénylène en C2-C10, le groupe alkylène ou alcénylène peut être substitué par un groupe alkyle en C1-C3 ou un groupe hydroxyle ; m est un entier de 0 à 5 ; n, qui est un nombre moyen de moles ajoutées du groupe oxyéthylène, est un entier de 2 à 150 ;

[Formule 4]     $CH_2=CR^3-CO-M^1$

dans la formule 4, $R^3$ est un atome d'hydrogène ou un groupe méthyle ; et $M^1$ est - $OM^2$ ou -$N(M^2)_2$ ; $M^2$, dont chaque occurrence peut être identique ou différente, est un atome d'hydrogène ou un groupe alkyle en C1-C3 ; et

[Formule 5]     $[M^{2+}_{1-x}N^{3+}_x(OH)_2][A^{n-}]_{x/n} \cdot yH_2O$

dans la formule 5, $M^{2+}$ est un cation métallique divalent ; $N^{3+}$ est un cation métallique trivalent ; A est une espèce chimique anionique qui a une liaison ionique entre des couches de la structure lamellaire de l'hydroxyde et a un nombre de charge égal à n ; x est un nombre qui est supérieur à 0 et inférieur à 1 ; et y est un nombre positif qui est supérieur à 0.

2. Adjuvant pour ciment selon la revendication 1, dans lequel le motif structural (b) comprend deux motifs structuraux ou plus dérivés de deux monomères acryliques ou plus représentés par la formule 4 ci-dessus.

3. Adjuvant pour ciment selon la revendication 2, dans lequel dans au moins un des deux monomères acryliques ou plus représentés par la formule 4, $M^1$ est -$OM^2$.

4. Adjuvant pour ciment selon la revendication 1 ou 2, dans lequel un rapport molaire du motif structurel (a) au motif structural (b) est dans une plage de 10/90 à 30/70.

5. Adjuvant pour ciment selon la revendication 1 ou 2, dans lequel $M^{2+}$ est sélectionné à partir du groupe constitué de $Mg^{2+}$, $Ca^{2+}$, $Co^{2+}$, $Cu^{2+}$, $Ni^{2+}$ et $Zn^{2+}$, $N^{3+}$ est sélectionné à partir du groupe constitué de $Al^{3+}$, $Cr^{3+}$, $Fe^{3+}$, $Ga^{3+}$, $In^{3+}$, $V^{3+}$ et $Ti^{3+}$, et $A^{n-}$ est sélectionné à partir du groupe constitué de $NO_2^-$, $NO_3^-$, $CO_3^{2-}$, $OH^-$, $O^{2-}$, $SO_4^{2-}$, un halogénure, un métalate et un anion d'acide organique.

6. Adjuvant pour ciment selon la revendication 1 ou 2, dans lequel la quantité du copolymère à base d'acide polycarboxylique (A) est dans une plage de 40 à 95 % en poids et la quantité de l'hydroxyde double lamellaire (B) est dans une plage de 5 à 60 % en poids sur la base d'un poids total de l'adjuvant pour ciment.

7. Procédé de préparation d'un adjuvant pour ciment, le procédé comprenant :

(I) la préparation (a) d'un composé macromonomère représenté par la formule 1 ci-dessous en mettant à réagir (a1) un composé représenté par la formule 2 ci-dessous avec (a2) un acide dicarboxylique saturé ou insaturé représenté par la formule 3, un anhydride de celui-ci ou un mélange de ceux-ci ;
(II) la préparation (A) d'un copolymère à base d'acide polycarboxylique en mettant à réagir (a) le composé macromonomère avec (b) un monomère acrylique représenté par la formule 4 ci-dessous ;
(III) la préparation (B) d'un hydroxyde double lamellaire représenté par la formule 5 ci-dessous et ayant une structure lamellaire ; et
(IV) la réaction (A) du copolymère à base d'acide polycarboxylique avec (B) l'hydroxyde double lamellaire :

[Formule 1]     $CH_2=CR^1-(CH_2)_m-O-(CH_2CH_2O)_n-CO-R^2-COOH$

dans lequel dans la formule 1, $R^1$ est un atome d'hydrogène ou un groupe méthyle ; $R^2$ est un groupe alkylène en C2-C10 ou un groupe alcénylène en C2-C10, le groupe alkylène ou alcénylène peut être substitué par un groupe alkyle en C1-C3 ou un groupe hydroxyle ; m est un entier de 0 à 5 ; et n, qui est un nombre moyen de moles ajoutées du groupe oxyéthylène, est un entier de 2 à 150 ;

[Formule 2]     $CH_2=CR^1-(CH_2)_m-O-(CH_2CH_2O)_n-H$

dans la formule 2, $R^1$, m et n sont tels que définis ci-dessus en référence à la formule 1 ;

[Formule 3]     HOOC-$R^2$-COOH

dans la formule 3, $R^2$ est tel que défini ci-dessus en référence à la formule 1 ;

[Formule 4]     $CH_2$=$CR^3$-CO-$M^1$

dans la formule 4, $R^3$ est un atome d'hydrogène ou un groupe méthyle ; et $M^1$ est - $OM^2$ ou -$N(M^2)_2$ ; et $M^2$, dont chaque occurrence peut être identique ou différente, est un atome d'hydrogène ou un groupe alkyle en C1-C3 ; et

[Formule 5]     $[M^{2+}_{1-x}N^{3+}_x(OH)_2][A^{n-}]_{x/n} \cdot yH_2O$

dans la formule 5, $M^{2+}$ est un cation métallique divalent ; $N^{3+}$ est un cation métallique trivalent ; A est une espèce chimique anionique qui a une liaison ionique entre des couches de la structure lamellaire de l'hydroxyde et un nombre de charge égal à n ; x est un nombre qui est supérieur à 0 et inférieur à 1 ; et y est un nombre positif qui est supérieur à 0.

8. Procédé selon la revendication 7, dans lequel à l'étape (I), le composant (a2) est l'anhydride d'acide dicarboxylique saturé ou insaturé représenté par la formule 3, et l'étape (I) est réalisée en l'absence d'un solvant et d'un additif dans des conditions atmosphériques tout en agitant à une température de 55 à 70 °C.

9. Procédé selon la revendication 7, dans lequel à l'étape (I), le composant (a2) est l'acide dicarboxylique saturé ou insaturé représenté par la formule 3, et l'étape (I) est réalisée en présence d'un inhibiteur de polymérisation tout en agitant à une température de 75 à 95 °C.

10. Procédé selon l'une quelconque des revendications 7 à 9, dans lequel l'étape (II) est réalisée en présence d'un initiateur de polymérisation, et l'initiateur de polymérisation est un initiateur à base de peroxyde.

FIG. 1

FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1195825

- EP 2412689 A2

**Non-patent literature cited in the description**

- **ISHII et al.** *Polymer Journal,* 2005, vol. 37 (3), 221-228

- **PLANK et al.** *Materials Letters,* 2006, vol. 60 (29-30), 3614-2617